Europäisches Patentamt
European Patent Office
Office européen des brevets

(19) (11) Veröffentlichungsnummer: **0 325 802 A1**

(12) **EUROPÄISCHE PATENTANMELDUNG**

(21) Anmeldenummer: 88121880.4

(22) Anmeldetag: 30.12.88

(51) Int. Cl.4: C07D 235/06 , C07D 235/08 , C07D 235/26 , C07D 235/10 , A61K 31/415

(30) Priorität: 05.01.88 DE 3800096

(43) Veröffentlichungstag der Anmeldung: 02.08.89 Patentblatt 89/31

(84) Benannte Vertragsstaaten: AT BE CH DE ES FR GB GR IT LI NL SE

(71) Anmelder: BAYER AG

D-5090 Leverkusen 1 Bayerwerk(DE)

(72) Erfinder: Bonse, Gerhard, Dr.
Wolfskaul 3
D-5000 Köln 80(DE)
Erfinder: Hallenbach, Werner, Dr.
Kleiststrasse 10
D-4018 Langenfeld(DE)
Erfinder: Lindel, Hans, Dr.
Carl-Duisberg-Strasse 321
D-5090 Leverkusen 1(DE)
Erfinder: Berschauer, Friedrich, Prof. Dr.
Claudiusweg 9
D-5600 Wuppertal 1(DE)

# (54) Verwendung von Benzimidazolderivaten als Leistungsförderer.

(57) Die vorliegende Erfindung betrifft die Verwendung von Benzimidazolderivaten der Formel

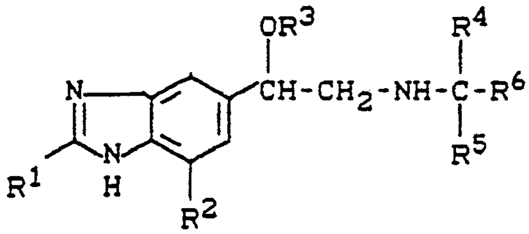

(I)

in welcher
$R^1$ für Wasserstoff, Hydroxy, Alkyl, Halogenalkyl steht,
$R^2$ für Wasserstoff, Alkyl, Halogen, CN, Halogenalkyl steht,
$R^3$ für Wasserstoff, Alkyl, Acyl steht,
$R^4$ für Wasserstoff, Alkyl, Halogenalkyl steht,
$R^5$ für Alkyl, Halogenalkyl steht,
$R^6$ für Alkyl, Cycloalkyl, gegebenenfalls substituiert durch Halogen, Cycloalkyl, OH, Alkoxy, Halogenalkoxy, Aryl, das seinerseits substituiert sein kann durch Alkyl, Halogen, CN oder die Reste $-COR^7$, $-O$-Alkylen-$COR^7$, -Alkylen-$R^8$ oder -O-Alkylen-$R^8$, steht,
$R^7$ für Hydroxy, Alkoxy oder $-NR^9R^{10}$ steht,
$R^8$ für Hydroxy, Alkoxy oder $-NR^9R^{10}$ steht,
$R^9$ für Wasserstoff, Alkyl steht,
$R^{10}$ für Wasserstoff, Alkyl steht

EP 0 325 802 A1

sowie deren Tautomere and physiologisch verträgliche Salze als Leistungsförderer für Tiere, neue Benzimidazolderivate, Zwischenprodukte und Verfahren zu ihrer Herstellung.

## Verwendung von Benzimidazolderivaten als Leistungsförderer

Die vorliegende Erfindung betrifft die Verwendung von Benzimidazolderivaten als Leistungsförderer für Tiere, neue Benzimidazolderivate sowie Zwischenprodukte und Verfahren zu ihrer Herstellung.

1-(5-Benzimidazolyl)-2-aminoethanol und -2-isopropylaminoethanol sind bekannt (GB-P 1 559 915). Es ist jedoch nichts bekannt über ihre Eignung als Leistungsförderer für Tiere.

Gegenstand der vorliegenden Erfindung ist:

1. Verwendung von Benzimidazolderivaten der Formel I

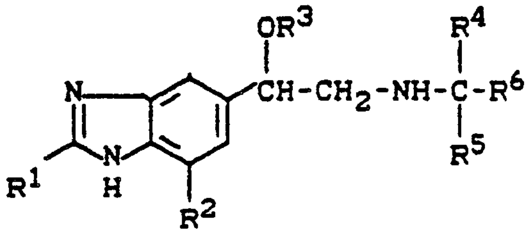

(I)

in welcher

$R^1$ für Wasserstoff, Hydroxy, Alkyl, Halogenalkyl steht,

$R^2$ für Wasserstoff, Alkyl, Halogen, CN, Halogenalkyl steht,

$R^3$ für Wasserstoff, Alkyl, Acyl steht,

$R^4$ für Wasserstoff, Alkyl, Halogenalkyl steht,

$R_5$ für Alkyl, Halogenalkyl steht,

$R^6$ für Alkyl, Cycloalkyl, gegebenenfalls substituiert durch Halogen, Cycloalkyl, OH, Alkoxy, Halogenalkoxy, Aryl, das seinerseits substituiert sein kann durch Alkyl, Halogen, CN oder die Reste $-COR^7$, $-O-Alkylen-COR^7$, $-Alkylen-R^8$ oder $-O-Alkylen-R^8$, steht,

$R^7$ für Hydroxy, Alkoxy oder $-NR^9R^{10}$ steht,

$R^8$ für Hydroxy, Alkoxy oder $-NR^9R^{10}$ steht,

$R^9$ für Wasserstoff, Alkyl steht,

$R^{10}$ für Wasserstoff, Alkyl steht

sowie deren Tautomere and physiologisch verträgliche Salze zur Förderung der Leistung und des Wachstums von Tieren.

2. Neue Benzimidazolderivate der Formel I

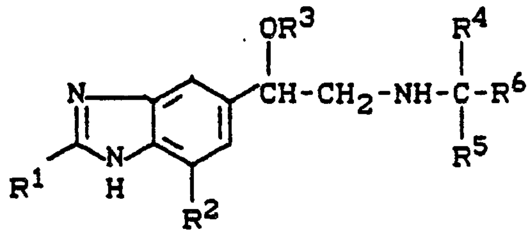

(I)

in welcher

$R^1$ für Wasserstoff, Hydroxy, Alkyl, Halogenalkyl steht,

$R^2$ für Wasserstoff, Alkyl, Halogen, CN, Halogenalkyl steht,

$R^3$ für Wasserstoff, Alkyl, Acyl steht,

$R^4$ für Wasserstoff, Alkyl, Halogenalkyl steht,

$R_5$ für Alkyl, Halogenalkyl steht,

$R^6$ für Alkyl, Cycloalkyl, gegebenenfalls substituiert durch Halogen, Cycloalkyl, OH, Alkoxy, Halogenalkoxy, Aryl, das seinerseits substituiert sein kann durch Alkyl, Halogen, CN oder die Reste $-COR^7$, $-O-Alkylen-COR^7$, $-Alkylen-R^8$ oder $-O-Alkylen-R^8$, steht,

$R^7$ für Hydroxy, Alkoxy oder $-NR^9R^{10}$ steht,

$R^8$ für Hydroxy, Alkoxy oder $-NR^9R^{10}$ steht,

$R^9$ für Wasserstoff, Alkyl steht,

$R^{10}$ für Wasserstoff, Alkyl steht
sowie deren Tautomere and physiologisch verträgliche Salze, ausgenommen Verbindungen in denen
$R^1$ für Wasserstoff steht,
$R^4$ für Wasserstoff steht,
$R^5$ für Methyl steht,
$R^6$ für Methyl steht,
sowie deren Tautomere and Salze.

3. Verfahren zur Herstellung der neuen Benzimidazolderivate der Formel I

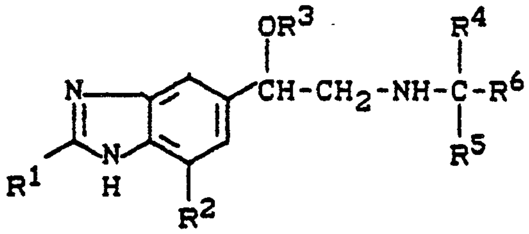

(I)

in welcher
$R^1$ für Wasserstoff, Hydroxy, Alkyl, Halogenalkyl steht,
$R^2$ für Wasserstoff, Alkyl, Halogen, CN, Halogenalkyl steht,
$R^3$ für Wasserstoff, Alkyl, Acyl steht,
$R^4$ für Wasserstoff, Alkyl, Halogenalkyl steht,
$R_5$ für Alkyl, Halogenalkyl steht,
$R^6$ für Alkyl, Cycloalkyl, gegebenenfalls substituiert durch Halogen, Cycloalkyl, OH, Alkoxy, Halogenalkoxy, Aryl, das seinerseits substituiert sein kann durch Alkyl, Halogen, CN oder die Reste $-COR^7$, $-O-Alkylen-COR^7$, $-Alkylen-R^8$ oder $-O-Alkylen-R^8$, steht,
$R^7$ für Hydroxy, Alkoxy oder $-NR^9R^{10}$ steht,
$R^8$ für Hydroxy, Alkoxy oder $-NR^9R^{10}$ steht,
$R^9$ für Wasserstoff, Alkyl steht,
$R^{10}$ für Wasserstoff, Alkyl steht
sowie deren Tautomere and physiologisch verträgliche Salze, ausgenommen Verbindungen in denen $R^1$ und $R^4$ für Wasserstoff sowie $R^5$ und $R^6$ für Methyl stehen, dadurch gekennzeichnet, daß man

a) Halogenmethylketone der Formel II

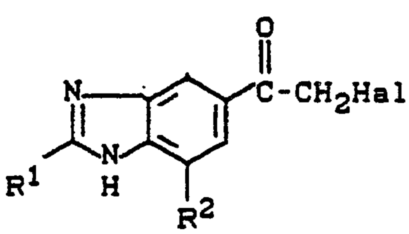

(II)

in welcher
$R^1$ und $R^2$ die oben angegebene Bedeutung haben und
Hal für Halogen steht,
mit Aminen der Formel III

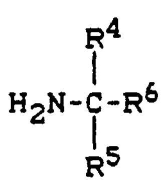

(III)

in welcher

$R^4$ bis $R^6$ die oben angegebene Bedeutung haben, umsetzt und anschließend die Carbonylgruppe reduziert oder

b) β-Halogenethylverbindungen der Formel IV

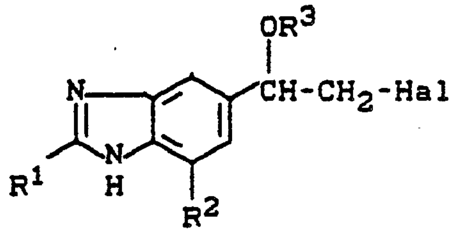

(IV)

in welcher
$R^1$ und $R^3$ die oben angegebene Bedeutung haben und
Hal für Halogen steht,
mit Aminen der Formel III

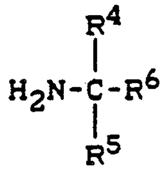

(III)

in welcher
$R^4$ bis $R^6$ die oben angegebene Bedeutung haben
umsetzt oder

c) für den Fall, daß in Formel I $R^4$ für Wasserstoff steht, indem man Verbindungen der Formel V

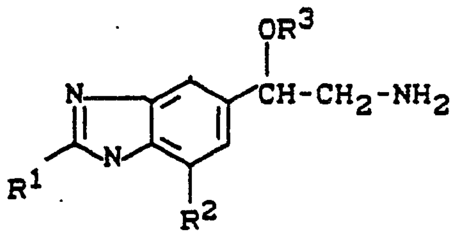

(V)

in welcher
$R^1$ und $R^3$ die oben angegebene Bedeutung haben,
mit Verbindungen der Formel VI

$R^5-\overset{\overset{O}{\|}}{C}-R^6$ (VI)

in welcher
$R^5$, $R^6$ die oben angegebene Bedeutung haben, unter reduzierenden Bedingungen umsetzt oder

d) Verbindungen der Formel VII

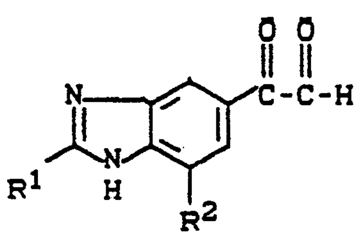

(VII)

in welcher
$R^1$ und $R^2$ die oben angegebene Bedeutung haben,
mit Aminen der Formel III

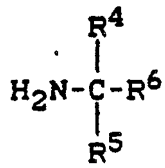

(III)

in welcher
$R^4$ bis $R^6$ die oben angegebene Bedeutung haben, unter reduzierenden Bedingungen umsetzt.

4. Neue Verbindungen der Formel II

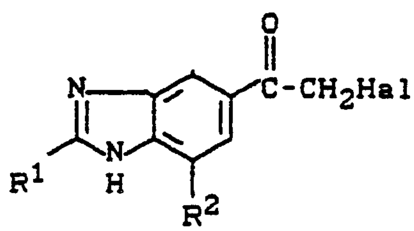

(II)

in welcher
$R^1$ für Wasserstoff
$R^2$ für Wasserstoff, Alkyl, Halogen
Hal für Halogen steht.

5. Verfahren zur Herstellung der neuen Verbindungen der Formel II gemäß (4),dadurch gekennzeichnet, daß man Acetylverbindungen der Formel VIII

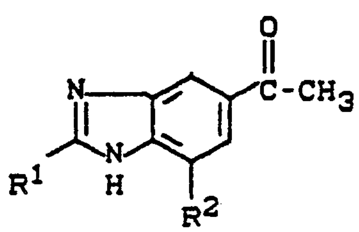

(VIII)

in welcher
$R^1$ für Wasserstoff
$R^2$ für Wasserstoff, Alkyl, Halogen steht
a) mit elementarem Halogen umsetzt, oder
b) mit Kupferhalogeniden der Formel $CuHal_2$ umsetzt.

6. Neue Verbindungen der Formel VIII

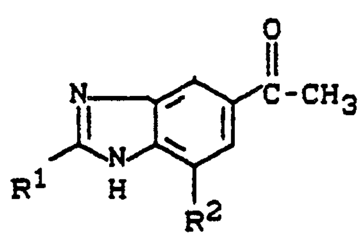

(VIII)

in welcher
$R^1$ für Wasserstoff steht,
$R^2$ für Wasserstoff, Alkyl oder Halogen steht.

7. Verfahren zur Herstellung der Verbindungen der Formel VIII gemäß (6), dadurch gekennzeichnet, daß man 3,4-Diaminoacetophenone der Formel IX

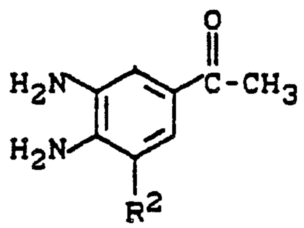

(IX)

in welcher
$R^2$ für Wasserstoff, Alkyl oder Halogen steht,
mit Ameisensäure in Gegenwart anorganischer Säuren umsetzt.

8. Neue Verbindungen der Formel IV

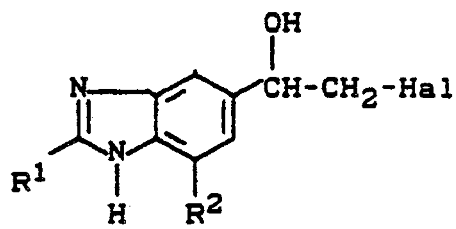

(IV)

in welcher
$R^1$ für Wasserstoff, Hydroxy, Alkyl, Halogenalkyl steht,
$R^2$ für Wasserstoff, Alkyl, Halogen, CN, Halogenalkyl steht.

9. Verfahren zur Herstellung der Verbindungen der Formel IV gemäß (8) dadurch gekennzeichnet, daß man Verbindungen der Formel II

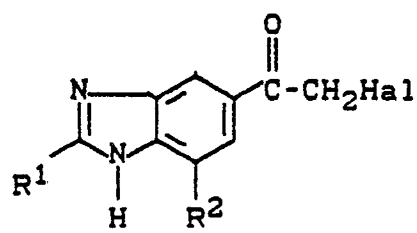

(II)

in welcher
$R^1$ und $R^2$ die bei (8) angegebenen Bedeutungen haben,
reduziert.

10. Neue Verbindungen der Formel V

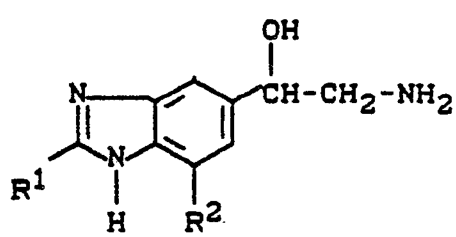

(V)

in welcher
$R^1$ für Wasserstoff, Alkyl, Halogenalkyl steht,
$R^2$ für Alkyl, Halogen, CN, Halogenalkyl steht.

11. Verfahren zur Herstellung der Verbindungen der Formel V gemäß (10), dadurch gekennzeichnet, daß man Verbindungen der Formel X

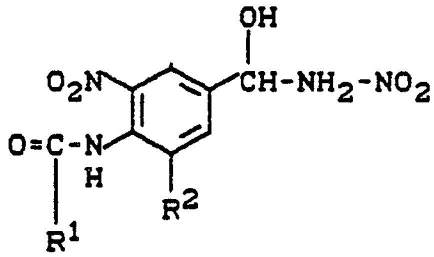

(X)

in welcher
$R^1$ und $R^2$ die bei 10 (oben) angegebene Bedeutung haben,
in Gegenwart von Katalysatoren mit Wasserstoff reduziert.

12. Neue Verbindungen der Formel VII

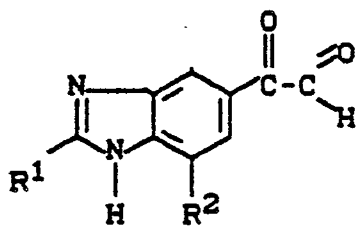

(VII)

in welcher
$R^1$ für Wasserstoff, Hydroxy, Alkyl, Halogenalkyl steht,
$R^2$ für Wasserstoff, Alkyl, Halogen, CN, Halogenalkyl steht.

13. Verfahren zur Herstellung der Verbindungen der Formel VII gemäß (12), dadurch gekennzeichnet, daß man

a) Verbindungen der Formel II

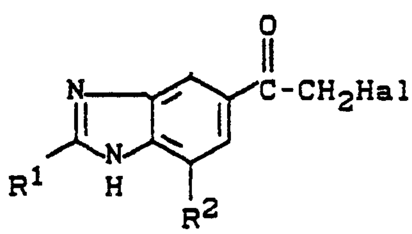

(II)

in welcher
$R^1$ und $R^2$ die bei (12) angegebene Bedeutung haben und
Hal für Halogen steht
oder

b) Verbindungen der Formel VIII

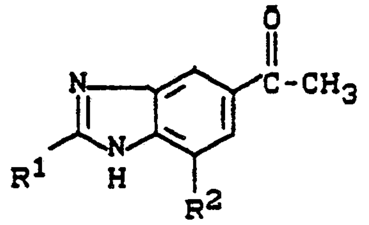

(VIII)

in welcher
$R^1$ und $R^2$ die bei (12) angegebene Bedeutung haben,
oxidiert.

Die Verbindungen der Formel I sind zum Teil bekannt. Die bekannten Verbindungen wirken bronchodilatorisch. Sie senken außerdem den Augeninnendruck.

Verbindungen der Formel I lassen sich als Leistungsförderer bei Tieren und besonders zur Förderung des Wachstums bei Tieren einsetzen.

Die Verbindungen der Formel I können auch in Form ihrer Racemate sowie als Gemische der zueinander diastereomeren oder enantiomeren Formen vorliegen.

Physiologisch verträgliche Salze der Verbindungen der Formel I können mit folgenden Säuren gebildet werden:
Salzsäure, Schwefelsäure, Phosphorsäure, Perchlorsäure, Brom-, Iodwasserstoffsäure, Salpetersäure, Essigsäure, Oxalsäure, Malonsäure, Bernsteinsäure, Ascorbinsäure, Äpfelsäure, Weinsäure, Maleinsäure, Fumarsäure, Methansulfonsäure, Benzoesäure, substituierte Benzoesäuren, Ameisensäure, Toluolsulfonsäure, Benzolsulfonsäure, Phthalsäure, Naphthalinsulfonsäure, Nikotinsäure, Palmitinsäure, Embonsäure.

Bevorzugt sind Verbindungen der Formel I,
in welcher
$R^1$ für H, OH steht,
$R^2$ für H, Halogen, CN, Halogenalkyl steht,
$R^3$ für H steht,
$R^4$ für H, $C_{1-4}$ Alkyl steht,
$R^5$ für $C_{1-4}$-Alkyl steht,
$R^6$ für $C_{1-4}$-Alkyl, gegebenenfalls substituiert durch Halogen, OH, $C_{1-4}$-Alkoxy, $C_{1-4}$-Halogenalkoxy, Phenyl, welches gegebenenfalls durch $C_{1-4}$-Alkyl, $COR^7$, -O-$C_{1-3}$-Alkylen-$COR^7$, $C_{1-3}$-Alkylen-$R^8$, O-$C_{1-3}$-Alkylen-$R^8$ substituiert ist, steht,
$R^7$ für OH, $C_{1-4}$-Alkoxy, Amino, Methylamino, Dimethylamino, steht,
$R^8$ für OH, $C_{1-4}$-Alkoxy, Amino, Methylamino, Dimethylamino steht.

Besonders bevorzugt sind Verbindungen der Formel I,
in welcher
$R^1$ für Wasserstoff oder OH steht,
$R^2$ für Wasserstoff, Chlor, Brom, Trifluormethyl steht,
$R^3$ für Wasserstoff steht,
$R^4$ für Wasserstoff oder Methyl steht,
$R^5$ für Methyl steht,
$R^6$ für Methyl steht, das gegebenenfalls substituiert ist durch OH oder Phenyl, das seinerseits gegebenenfalls substituiert ist durch Methyl, Ethyl, Methoxyethyl, Hydroxyethyl, Methoxyethyloxy, Hydroxyethyloxy.

Im einzelnen seien neben den Beispielen die folgenden Verbindungen der Formel I genannt:

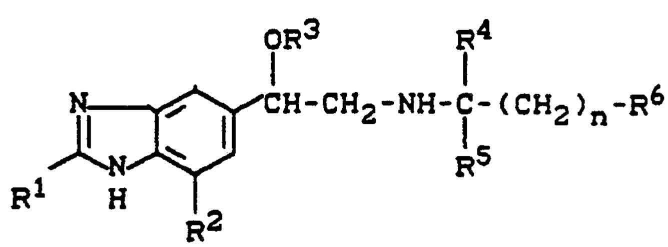

| $R^1$ | $R^2$ | $R^3$ | $R^4$ | $R^5$ | n | $R^6$ |
|---|---|---|---|---|---|---|
| H | H | H | H | $CH_3$ | 1 | H |
| H | H | H | $CH_3$ | $CH_3$ | 1 | OH |
| OH | H | H | H | $CH_3$ | 1 | H |
| OH | Cl | H | $CH_3$ | $CH_3$ | 1 | H |
| H | H | H | H | $CH_3$ | 1 | $C_6H_5$ |

Die neuen Verbindungen der Formel I lassen sich nach den unter 3 a - d angegebenen Verfahren herstellen.

Setzt man bei Verfahren 3a) als Verbindung der Formel II 4-Chlorbenzimidazoyl-6-brommethylketon und als Amin der Formel III tert.-Butylamin ein, läßt sich Verfahren 3a) durch folgendes Schema wiedergeben:

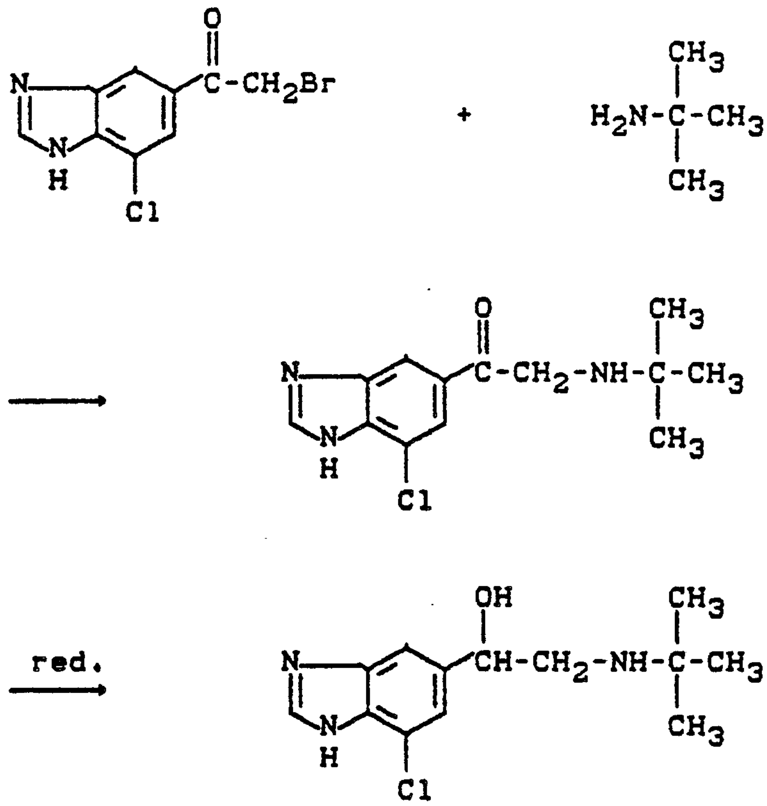


Die Verbindungen der Formel II sind teilweise neu. Ihre Herstellung wird weiter unten beschrieben. Die Substituenten $R^1$ und $R^2$ besitzen bevorzugt die weiter oben angegebenen bevorzugten Bedeutungen. Im einzelnen seien folgende Verbindungen der Formel II genannt:

Benzimidazoyl-5-brommethylketon
Benzimidazoyl-5-chloromethylketon
4-Brombenzimidazoyl-6-brommethylketon
4-Brombenzimidazoyl-6-chlormethylketon

Die Amine der Formel III sind bekannt (vgl. z.B. EP-OS 23.385). Die Substituenten $R^4$ bis $R^6$ besitzen bevorzugt die weiter oben bei den Verbindungen der Formel I genannten bevorzugten Bedeutungen. Im einzelnen seien folgende Amine der Formel III genannt:

Isopropylamin
Isobutylamin
sec. Butylamin
tert. Butylamin
2-Amino-2-methyl-propanol-1
3-(4-Carbomethoxyphenyl)-2-propylamin,
3-(4-Methoxycarbonylmethoxyphenyl)-2-propylamin,
3-(4-Carboxyphenyl)-2-propylamin,

3-(4-Carboxymethoxyphenyl)-2-propylamin,
3-(4-Hydroxymethylphenyl)-2-propylamin,
3-(4-Dimethylaminomethylphenyl)2-propylamin,
3-(4-(2-Hydroxyethyl)phenyl)-2-propylamin.

Als Reduktionsmittel zur Durchführung des Verfahrens 3a) seien folgende Reduktionsmittel genannt: $H_2$/Katalysator, als Katalysator seien beispielsweise genannt: $PtO_2$, Pd-Aktivkohle;
komplexe Metallhydride wie z.B. $LiAlH_4$, $NaBH_4$, $NaBH_3CN$.

Besonders bevorzugt werden folgende Reduktionsmittel eingesetzt:
$NaBH_4$ und $NaBH_3CN$

Verfahren 3a) wird durchgeführt, indem man die Verbindungen II und III in einem Verdünnungsmittel in etwa äquimolarem Verhältnis zusammengibt und anschließend reduziert.

Die Reduktion wird bei Temperaturen von -20 °C bis +100 °C bevorzugt durchgeführt.

Es wird bevorzugt bei Normaldruck gearbeitet.

Als Verdünnungsmittel dienen alle inerten organischen Lösungsmittel. Hierzu gehören insbesondere aliphatische und aromatische Kohlenwasserstoffe wie Pentan, Hexan, Cyclohexan, Petrolether, Ligroin, Benzol, Toluol; chlorierte Kohlenwasserstoffe wie Methylenchlorid, Ethylenchlorid, Chloroform; Ether wie Diethylether und Glykoldimethylether; Nitrile wie Acetonitril, Propionitril und Benzonitril; Alkohole wie Methanol, Ethanol, n- und i-Propanol.

Bevorzugt sind Alkohole, wobei ohne Isolierung der Zwischenstufen sofort die Reduktion durchgeführt werden kann.

Setzt man bei Verfahren 3b) als Verbindunge der Formel IV 1-(5-Benzimidazoyl)-1-hydroxy-2-chlorethan und als Amin der Formel III 3-(4-Carbomethoxyphenyl)-2-propylamin ein, läßt sich das Verfahren 3b) durch folgendes Schema wiedergeben:

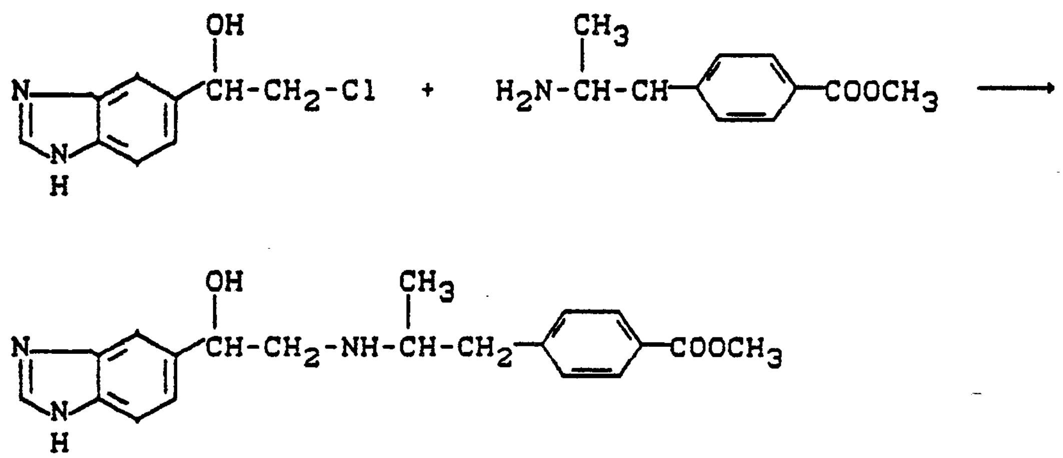

β-Halogenethylverbindungen der Formel IV sind neu. Ihre Herstellung wird weiter unten beschrieben. Die Substituenten $R^1$ bis $R^3$ besitzen bevorzugt die bei den Verbindungen der Formel I angegebenen bevorzugten Bedeutungen. Im einzelnen seien folgende Verbindungen der Formel IV genannt:
1-(5-Benzimidazoyl)-2-chlorethanol
1-(5-Benzimidazoyl)-2-bromethanol
1-(4-Brom-6-benzimidazoyl)-2-chlorethanol
1-(4-Brom-6-benzimidazoyl)-2-bromethanol
1-(4-Chlor-6-benzimidazoyl)-2-bromethanol

Verfahren 3b) wird durchgeführt indem man die β-Halogenethylverbindung der Formel IV mit überschüssigem Amin der Formel III gegebenenfalls in Gegenwart eines Verdünnungsmittels umsetzt.

Die Reaktion wird bei Temperaturen von +20 bis +150 °C durchgeführt.

Es wird bei Normaldruck oder unter erhöhtem Druck gearbeitet.

Als Verdünnungsmittel dienen alle inerten organischen Lösungsmittel. Hierzu gehören insbesondere aliphatische und aromatische, gegebenenfalls halogenierte Kohlenwasserstoffe, wie Pentan, Hexan, Cyclohexan, Benzol, Toluol, Methylenchlorid, Chloroform ferner Ether wie Diethylether, Tetrahydrofuran und Dioxan, weiterhin Nitrile wie Acetonitril und Benzonitril, ferner Amide wie Dimethylformamid, ferner Alkohole wie Methanol, Ethanol, n- und 1-Propanol.

Bevorzugt eingesetzt werden Alkohole.

Setzt man bei Verfahren 3c) als Verbindung der Formel V 1-(5-Benzimidazoyl)-2-aminoethanol und als Verbindung der Formel VI 4-(Dimethylaminomethyl)-phenylaceton ein, läßt sich Verfahren 3c) durch folgendes Reaktionsschema wiedergeben:

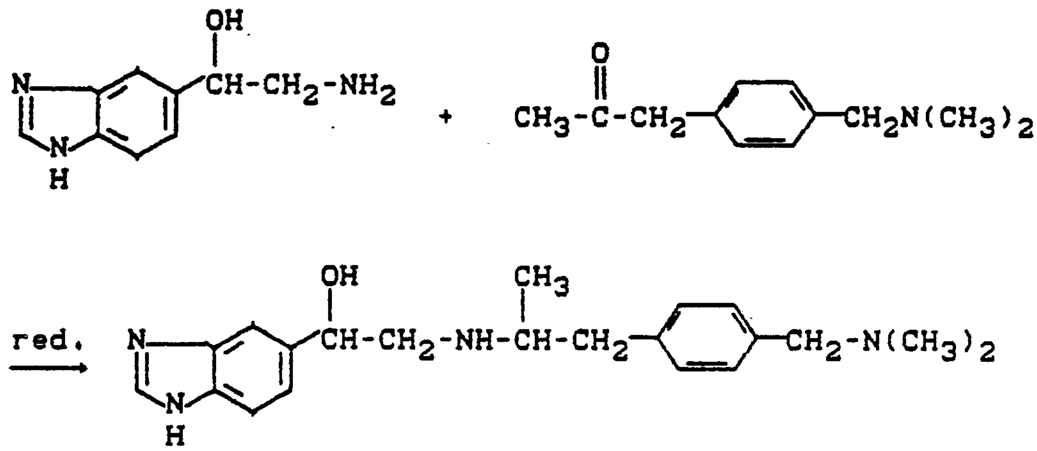


Die Verbindungen der Formel V sind teilweise bekannt (vgl. z. B. DE-OS 2 754 148). Neue Verbindungen der Formel V werden weiter unten beschrieben. Die Substituenten $R^1$ bis $R^3$ besitzen bevorzugt die weiter oben bei den Verbindungen der Formel I angegebenen bevorzugten Bedeutungen. Im einzelnen seien folgende Verbindungen der Formel V genannt.

1-(4-Brom-6-benzimidazoyl)-2-aminoethanol

1-(4-Chlor-6-benzimidazoyl)-2-aminoethanol

Verbindungen der Formel VI sind bekannt (vgl. z.B. EP-OS 70 133). Die Substituenten $R^5$ und $R^6$ besitzen bevorzugt die weiter oben bei den Verbindungen der Formel I angegebenen Bedeutungen.

Im einzelnen seien folgende Verbindungen der Formel VI genannt:

Aceton

Methylethylketon

Methylisobutylketon

4-Carbomethoxyphenylaceton,

4-(Carbomethoxymethoxy)phenylaceton

4-(2-Hydroxyethoxy)phenylaceton

Verfahren 3c) wird durchgeführt, indem man etwa äquimolare Mengen der Verbindungen der Formel V und VI in einem Verdünnungsmittel vorlegt und die Mischung reduziert.

Die Reaktion wird bei Temperaturen von 0 °C bis 150 °C durchgeführt.

Es wird bevorzugt bei Normaldruck gearbeitet.

Als Verdünnungsmittel dienen alle inerten organischen Lösungsmittel. Hierzu gehören aliphatische und aromatische, gegebenenfalls halogenierte Kohlenwasserstoffe wie Pentan, Hexan, Cyclohexan, Benzol, Toluol, Methylenchlorid, Ethylenchlorid, Chloroform, Chlorbenzol, ferner Ether wie Diethylether, Tetrahydrofuran, Dioxan, weiterhin Nitrile wie Acetonitril, Benzonitril, Amide wie Dimethylformamid, Alkohole wie Methanol, Ethanol.

Als Reduktionsmittel dienen:

$H_2$/Katalysator, als Katalysator sei beispielsweise $PtO_2$ genannt; komplexe Metallhydride wie z. B. $LiAlH_4$, $NaBH_4$, $NaBH_3CN$.

Setzt man bei Verfahren 3d) als Verbindung der Formel VII 5-Benzimidazoylglyoxal und als Amin der Formel III 3-(4-Methoxyphenyl)-2-propylamin ein, läßt sich Verfahren 3d) durch folgendes Reaktionsschema wiedergeben:

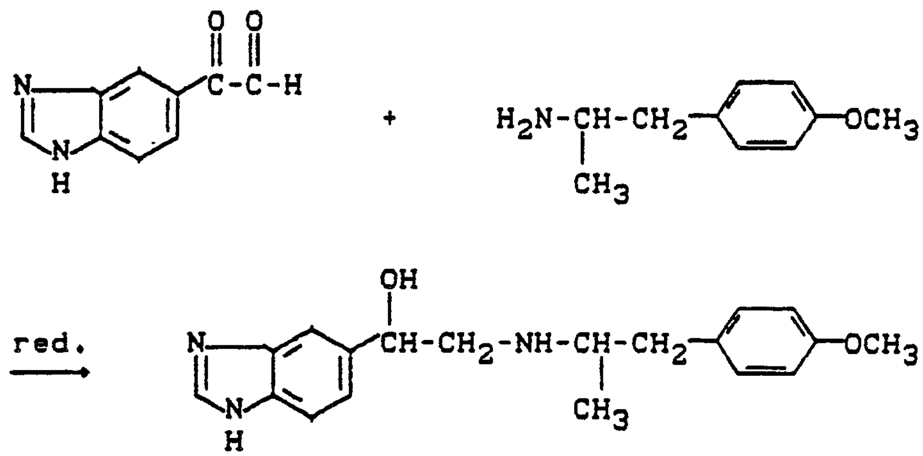


Verbindungen der Formel VII sind neu. Ihre Herstellung wird weiter unten beschrieben. Die Substituenten $R^1$ und $R^2$ besitzen bevorzugt die weiter oben bei den Verbindungen der Formel I angegeben bevorzugten Bedeutungen. Im einzelnen seien folgende Verbindungen der Formel VII genannt:

2-Hydroxy-5-benzimidazoylglyoxal
2-Hydroxy-4-brom-6-benzimidazoylglyoxal
2-Hydroxy-4-chlor-6-benzimidazoylglyoxal

Verfahren 3d) wird durchgeführt, indem man zur Verbindung der Formel VII in einem Verdünnungsmittel etwa die äquivalente Menge des Amins der Formel III zusetzt und anschließend reduziert.

Die Reaktion wird bei Temperaturen von 0° C bis 100° C durchgeführt.

Es wird bevorzugt bei Normaldruck gearbeitet.

Als Verdünnungsmittel dienen alle inerten organischen Lösungsmittel. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl- und Dibutylether, Glykoldimethylether und Diglykoldimethylether, Tetrahydrofuran und Dioxan, außerdem Ester, wie Essigsäuremethylester und -ethylester, ferner Nitrile, wie z. B. Acetonitril und Propionitril, Benzonitril, Glutarsäuredinitril, darüber hinaus Amide, wie z. B. Dimethylformamid, Dimethylacetamid und N-Methylpyrrolidon, sowie Tetramethylensulfon und Hexamethylphosphorsäuretriamid, weiterhin Alkohole wie Methanol, Ethanol, n- und i-Propanol.

Als Reduktionsmittel dienen
$H_2$/Katalysator, als Katalysator seien $PtO_2$ und Pd-Kohle genannt; ferner komplexe Metallhydride wie $LiAlH_4$ und $NaBH_4$.

Wie bereits erwähnt, lassen sich die neuen Verbindungen der Formel II nach den unter 5 angegebenen Verfahren herstellen.

Setzt man bei Verfahren 5a) als Verbindung der Formel VIII 5-Acetylbenzimidazol und als Halogen Hal Brom ein, läßt sich die Reaktion durch folgendes Schema darstellen:

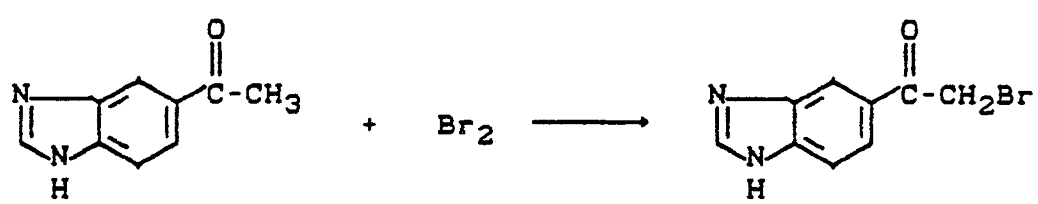

Die Verbindungen der Formel VIII sind neu. Ihre Herstellung wird weiter unten beschrieben.

Im einzelnen seien folgende Verbindungen der Formel VIII genannt:

5-Acetylbenzimidazol
4-Chlor-6-acetylbenzimidazol
4-Brom-6-acetylbenzimidazol

Verfahren 5a) wird durchgeführt, indem man zu Verbindung VIII in einem Verdünnungsmittel die äquivalente Menge Halogen, eventuell in einem Verdünnungsmittel gelöst, zugibt.

Die Reaktion wird bei +20° C bis +150° C durchgeführt, bevorzugt bei der Siedetemperatur des

verwendeten Verdünnungsmittels.

Es wird bevorzugt bei Normaldruck gearbeitet.

Als Verdünnungsmittel seien genannt: aliphatische, gegebenenfalls halogenierte Kohlenwasserstoffe wie Pentan, Hexan, Heptan, Cyclohexan, Methylenchlorid, Ethylenchlorid, Chloroform, Tetrachlorkohlenstoff, Alkohole wie Methanol, Ethanol, Ester wie Essigsäureethylester und Mischungen aus diesen Verdünnungsmitteln.

Setzt man bei Verfahren 5b) als Verbindung der Formel VIII 4-Chlor-6-acetylbenzimidazol und als Verbindung der Formel $CuHal_2$ Kupfer(II)bromid ein, läßt sich die Reaktion durch folgendes Schema darstellen:

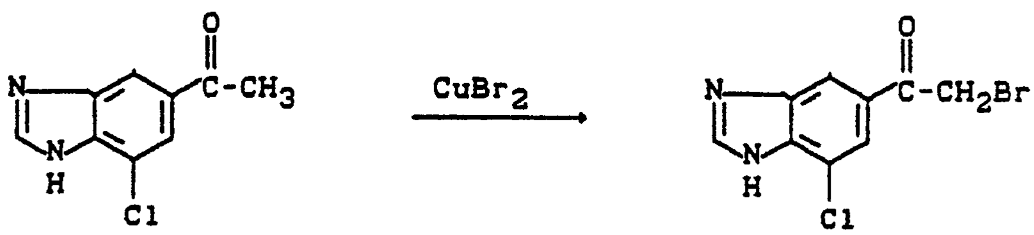


Verfahren 5b) wird durchgeführt, indem man äquivalente Mengen der Verbindung der Formel VIII und der Verbindung $CuHal_2$ im Verdünnungsmittel 1 - 24 h, bevorzugt 6 - 12 h unter Rückfluß erhitzt.

Die übrigen Reaktionsparameter sowie die Verdünnungsmittel sind bei Verfahren 5a) beschrieben.

Wie bereits erwähnt, lassen sich die neuen Verbindungen der Formel VIII nach dem unter 7 angegebenen Verfahren herstellen.

Setzt man bei dem Verfahren als Verbindung der Formel IX 3,4-Diaminoacetophenon ein, läßt es sich durch folgendes Schema wiedergeben:

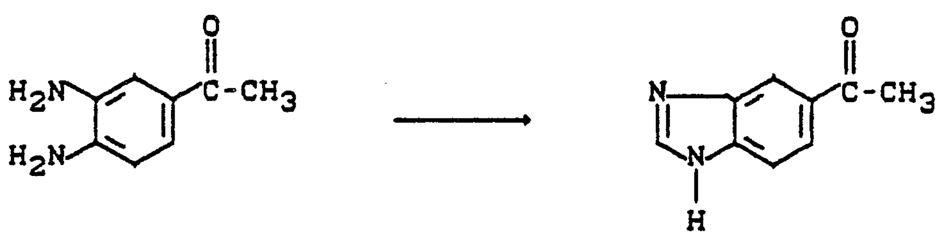

Verbindungen der Formel IX sind bekannt (JACS 80 (1958),1657) oder lassen sich analog zu bekannten Verfahren herstellen. Der Rest $R^2$ besitzt bevorzugt die Bedeutung Wasserstoff, $C_{1-4}$-Alkyl insbesondere Methyl, Chlor, Brom. Im einzelnen seien folgende Verbindungen der Formel IX genannt:

3-Brom-4,5-diaminoacetophenon

3-Chlor-4,5-diaminoacetophenon

Das Verfahren wird durchgeführt, indem man eine Verbindung der Formel IX in Gegenwart einer anorganischen Säure mit Ameisensäure umsetzt.

Zu den anorganischen Säuren zählen Halogenwasserstoffsäuren wie Salzsäure, Schwefelsäure, Phosphorsäure. Die Reaktion wird bei Temperaturen von +20 °C bis +120 °C durchgeführt.

Es wird bevorzugt in einer wässrigen Lösung der anorganischen Säure gearbeitet. Es wird bei Normaldruck gearbeitet.

Wie bereits erwähnt, lassen sich die neuen Verbindungen der Formel IV nach dem unter 9 angegenen Verfahren herstellen.

Setzt man bei Verfahren 9 als Verbindung der Formel II (2-Hydroxy-5-benzimidazoyl)chlormethylketon ein, läßt sich das Verfahren durch folgendes Reaktionsschema darstellen:

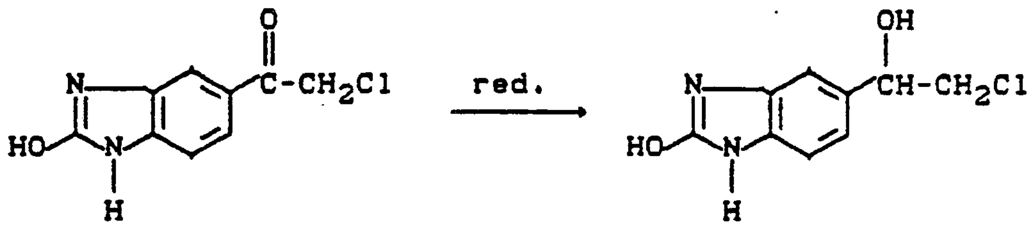

Die Substituenten $R^1$, $R^2$ und Hal in den Verbindungen der Formel II besitzen die weiter oben angegebenen bevorzugten Bedeutungen.

Als Reduktionsmittel zur Durchführung des Verfahrens seien genannt:

$H_2$/Katalysator, als Katalysator seien genannt: $PtO_2$, Pd/Kohle; komplexe Metallhydride, wie z.B. $LiAlH_4$, $NaBH_4$, $NaBH_3CN$. Bevorzugt werden $NaBH_4$ und $NaBH_3CN$ eingesetzt.

Das Verfahren wird durchgeführt, indem man die Verbindung II in einem Verdünnungsmittel mit dem Reduktionsmittel umsetzt.

Die Reaktion wird bei Temperaturen von -20 °C bis +100 °C durchgeführt.

Es wird bevorzugt bei Normaldruck gearbeitet.

Als Verdünnungsmittel dienen alle inerten organischen Lösungsmittel. Hierzu zählen insbesondere gegebenenfalls halogenierte aliphatische und aromatische Kohlenwasserstoffe wie Pentan, Hexan, Cyclohexan, Benzol, Toluol, Methylenchlorid, Chloroform, Chlorbenzol; Ether wie Diethylether, Tetrahydrofuran; Nitrile wie Acetonitril, Benzonitril, Alkohole wie Methanol, Ethanol, n- und i-Propanol. Bevorzugt werden Alkohole eingesetzt.

Wie bereits erwähnt sind die Verbindungen der Formel V zum Teil bekannt (z.B. aus GB-P 1 559 915). Die neuen Verbindungen der Formel V sind unter 10 (oben) zusammengefaßt. Ihre Herstellung erfolgt nach dem unter 11 angegebenen Verfahren. Das Verfahren wird analog zu dem in GB-P-1 559 915 beschriebenen Verfahren durchgeführt.

Wie bereits erwähnt, lassen sich die neuen Verbindungen der Formel VII nach den unter 13 angegebenen Verfahren herstellen.

Setzt man bei Verfahren 13a) als Verbindung der Formel II (4-Chlor-6-benzimidazoyl)chlormethylketon ein, läßt sich das Verfahren durch das folgende Schema wiedergeben:

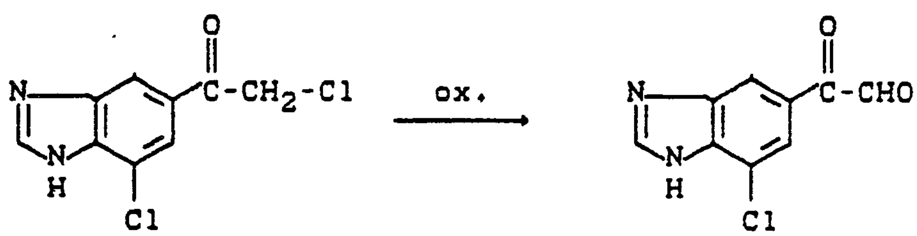

Als Halogenmethylketone der Formel II werden bevorzugt die weiter oben genannten Verbindungen eingesetzt.

Das Verfahren 13a) wird durchgeführt, indem man die Verbindungen der Formel II, gegebenenfalls in Gegenwart eines Verdünnungsmittels, oxidiert.

Die Reaktion wird bei Temperaturen von +20 °C bis +100 °C durchgeführt.

Es wird bevorzugt bei Normaldruck gearbeitet.

Als Oxidationsmittel wird bevorzugt Dimethylsulfoxid verwendet (N. Kornblum et. al., JACS 79, 6562 (1957)).

Wird die Umsetzung in Gegenwart eines Verdünnungsmittels durchgeführt, können alle inerten organischen Lösungsmittel verwendet werden. Hierzu gehören insbesondere aliphatische und aromatische, gegebenenfalls halogenierte Kohlenwasserstoffe wie Pentan, Hexan, Cyclohexan, Benzol, Toluol, Methylenchlorid, Chloroform, Chlorbenzol; Ether wie Diethylether, Tetrahydrofuran; Nitrile wie Acetonitril, Benzonitril. Bevorzugt wird in Dimethylsulfoxid ohne ein wieteres Lösungsmittel gearbeitet.

Setzt man bei Verfahren 13b) als Verbindung der Formel VIII 4-Brom-6-acetylbenzimidazol ein, läßt es sich durch folgendes Schema wiedergeben:

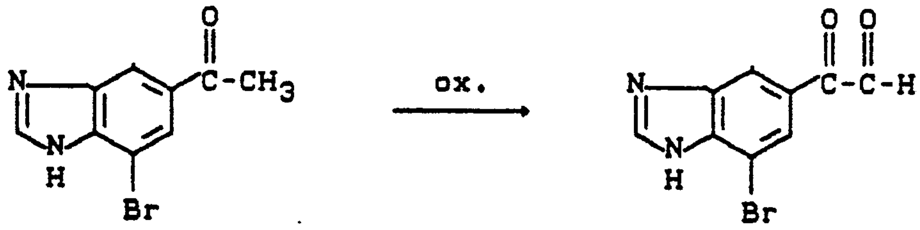

Das Verfahren wird durchgeführt, indem man die Verbindung der Formel VIII in Gegenwart eines Verdünnungsmittels mit Selendioxid oxidiert (vgl. Org. React. 5 (1949), 331).

Die Wirkstoffe eignen sich bei günstiger Warmblütertoxizität als Mittel zur Leistungsförderung bei Zucht-und Nutztieren. Sie dienen dabei zur Förderung und Beschleunigung des Wachstums, der Milch-und Wollproduktion, sowie zur Verbesserung der Futterverwertung, der Fleischqualität und zur Verschiebung des Fleisch-Fett-Verhältnisses zugunsten von Fleisch.

Zu den Nutz- und Zuchttieren gehören Säugetiere wie z. B. Rinder, Pferde, Schafe, Schweine, Ziegen, Kamele, Wasserbüffel, Esel, Kaninchen, Damwild, Rentiere, Pelztiere wie z. B. Nerze, Chinchilla, Waschbär, Vögel wie z. B. Hühner, Gänse, Puten, Enten, Süß- und Salzwasserfische wie z. B. Forellen, Karpfen, Aale.

Die Wirkstoffe werden unabhängig vom Geschlecht der Tiere während allen Wachstums- und Leistungsphasen der Tiere eingesetzt. Bevorzugt werden die Wirkstoffe während der intensiven Wachstums- und Leistungsphase eingesetzt. Die intensive Wachstums- und Leistungsphase dauert je nach Tierart von einem Monat bis zu 10 Jahren. Besonders wertvoll erweisen sich die Wirkstoffe bei Aufzucht und Haltung von Jung- und Masttieren.

Die Anwendung der Wirkstoffe erfolgt direkt oder in Form von für Tiere geeigneten Zubereitungen enteral oder parenteral. Die enterale Anwendung der Wirkstoffe geschieht z. B. oral in Form von Pulvern, Tabletten, Kapseln, Pasten, Tränken, Granulaten, Boli, über oral applizierbare Lösungen, Emulsionen oder Suspensionen sowie über das Futter oder über das Trinkwasser. Die parenterale Anwendung geschieht z. B. in Form der Injektion (intramuskulär, subcutan, intravenös oder durch Implantate).

Besonders hervorgehoben seien Zubereitungen zur Verabreichung über das Futter oder das Trinkwasser. Dabei können die Wirkstoffe dem Futter direkt oder in Form von Prämixen oder Futterkonzentraten zugesetzt werden.

Zum Futter zählen Einzelfuttermittel pflanzlicher Herkunft wie Heu, Rüben, Getreide und Getreidenebenprodukte, Melasse, Silage, Einzelfuttermittel tierischer Herkunft wie Fleisch, Fette, Milchprodukte, Knochenmehl, Fischprodukte, die Einzelfuttermittel wie Vitamine, Proteine, Zucker, Stärke, Mehle, Aminosäuren z. B. DL-Methionin, Salze wie Kalk und Kochsalz. Zum Futter zählen auch Ergänzungs-, Fertig- und Mischfuttermittel. Diese enthalten Einzelfuttermittel in einer Zusammensetzung, die eine ausgewogene Ernährung hinsichtlich der Energie- und Proteinversorgung sowie der Versorgung mit Vitaminen, Mineralsalzen und Spurenelementen sicherstellt.

Prämixe und Futterkonzentrate sind Mischungen des Wirkstoffs mit Trägerstoffen und gegebenenfalls wieteren Hilfsstoffen. Zu den Trägerstoffen zählen alle Einzelfuttermittel oder Gemische derselben.

Die Wirkstoffe können in den Zubereitungen allein oder in Mischungen mit anderen leistungsfördernden Wirkstoffen, mineralischen Futtermitteln, Spurenelement-Verbindungen, Vitaminen, stickstoffhaltigen Nicht-Protein-Verbindungen, Farbstoffen, Antioxidantien, Aromastoffen, Emulgatoren, Fließhilfsstoffen, Konservierungs- und Preßhilfsstoffen vorliegen.

Andere leistungsfördernde Wirkstoffe sind z. B. Antibiotika wie Tylosin und Virginamycin.

Mineralische Futtermittel sind z. B. Dicalciumphosphat, Magnesiumoxid, Natriumchlorid.

Spurenelement-Verbindungen sind z. B. Eisenfumarat, Natriumiodid, Kobaltchlorid, Kupfersulfat, Zinkoxid, Selenverbindungen.

Vitamine sind z. B. Vitamin A, Vitamin $D_3$, Vitamin E.

Stickstoffhaltige Nicht-Protein-Verbindungen sind z. B. Biuret, Harnstoff.

Farbstoffe sind z. B. Carotinoide wie Canthaxidin, Zeaxanthin, Capsanthin oder Farbstoffe, die zur Färbung von Lebensmitteln zugelassen sind.

Antioxidantien sind z. B. Ethoxyquin, Butylhydroxy-Toluol, Ascorbinsäure.

Aromastoffe sind z. B. Vanillin.

Emulgatoren sind z. B. Ester der Milchsäure, Lecithin.

Fleißhilfsstoffe sind z. B. Natriumstearat, Calciumstearat, Kieselsäuren, Bentonite, Ligninsulfonate.

Konservierungsstoffe sind z. B. Propionsäure, Calciumpropionat, Sorbinsäure, Ascorbinsäure.

Preßhilfsstoffe sind z. B. Ligninsulfonate, Celluloseether.

Die Konzentration der Wirkstoffe im Futter beträgt normalerweise etwa 0,001-500 ppm, bevorzugt 0,1-50 ppm.

Die Konzentration der Wirkstoffe in den Prämixen oder Futterkonzentraten beträgt ca. 0,5 bis 50 Gewichtsprozent, bevorzugt 1 bis 20 Gewichtsprozent.

Die Menge der Wirkstoffe, die den Tieren zur Erreichung des gewünschten Effektes verabreicht wird, kann wegen der günstigen Eigenschaften der Wirkstoffe weitgehend variiert werden. Sie liegt vorzugsweise bei etwa 0,001 bis 50 mg/kg insbesondere 0,01 bis 5 mg/kg Körpergewicht pro Tag. Die passende Menge des Wirkstoffs sowie die passende Dauer der Verabreichung hängen insbesondere von der Art, dem Alter, dem Geschlecht, dem Gesundheitszustand und der Art der Haltung und Fütterung der Tiere ab und sind durch jeden Fachmann leicht zu ermitteln.

Die Wirkstoffe werden den Tieren nach den üblichen Methoden verabreicht. Die Art der Verabreichung hängt insbesondere von der Art, dem Verhalten und dem Gesundheitszustand der Tiere ab.

Die Wirkstoffe können einmalig verabreicht werden. Die Wirkstoffe können aber auch während der ganzen oder während eines Teils der Wachstums- und Leistungsphase temporär oder kontinuierlich verabreicht werden. Bei kontinuierlicher Verabreichung kann die Anwendung ein-oder mehrmals täglich in regelmäßigen oder unregelmäßigen Abständen erfolgen.

Beispiel für die Zusammensetzung eines Kükenaufzuchtfutters, das den erfindungsgemäßen Wirkstoff enthält:

200 g Weizen, 340 g Mais, 361 g Sojaschrot, 60 g Rindertalg, 15 g Dicalciumphosphat, 10 g Calciumcarbonat, 4 g iodiertes Kochsalz, 7,5 g Vitamin-Mineral-Mischung der unten angegebenen Zusammensetzung und 2,5 g WirkstoffPrämix der unten angegebenen Zusammensetzung ergeben nach sorgfältigem Mischen 1 kg Futter.

In einem kg Vitamin-Mineral-Mischung sind enthalten: 600 I.E. Vitamin A, 100 I.E. Vitamin $D_3$, 10 mg Vitamin E, 1 mg Vitamin $K_3$, 3 mg Riboflavin, 2 mg Pyridoxin, 20 mcg Vitamin $B_{12}$, 5 mg Calciumpantothenat, 30 mg Nikotinsäure, 200 mg Cholinchlorid, 200 mg $MnSO_4 \times H_2O$, 140 mg $ZnSO_4 \times 7\ H_2O$, 100 mg $FeSO_4 \times 7\ H_2O$ und 20 mg $CuSO_4 \times 5\ H_2O$ in Getreidemehl als Trägerstoff.

1 kg Wirkstoff-Prämix enthalten 100 g Wirkstoff, 900 g Weizenmehl.

Beispiel für die Zusammensetzung eines Schweineaufzuchtfutters, das den erfindungsgemäßen Wirkstoff enthält:

630 g Futtergetreideschrot (zusammengesetzt aus 200 g Mais, 150 g Gerste-, 150 g Hafer- und 130 g Weizen schrot), 80 g Fischmehl, 60 g Sojaschrot, 60 g Tapiokamehl, 38 g Bierhefe, 50 g Vitamin-Mineral-Mischung (Zusammensetzung wie beim Kükenfutter) 30 g Leinkuchenmehl, 30 g Maiskleberfutter, 10 g Sojaöl, 10 g Zuckerrohrmelasse und 2 g Wirkstoff-Prämix ergeben nach sorgfältigem Mischen 1 kg Futter.
1 kg Wirkstoff-Prämix enthält 200 g Wirkstoff, 20 g Pflanzenöl, 780 g Calciumcarbonatpulver.

Beispiel für die Zusammensetzung eines Futters für Rinder, das den erfindungsgemäßen Wirkstoff enthält:

69,95 % Futtergetreideschrot, 10 % gemahlene Maiskolben, 8 % Sojabohnenmeht, 5 % Luzernemehl, 5 % Melasse, 0,6 % Harnstoff, 0,5 % Calciumphosphat, 0,5 % Calciumcarbonat, 0,3 % Kochsalz, 0,15 % Vitamin-Mineral-Mischung und 0,2 % Wirkstoff-Prämix der beim Schweineaufzuchtfutter angegebenen Zusammensetzung. Die Vitamin-Mineral-Mischung enthält pro kg 70.000 i.E. Vitamin A, 70.000 Vitamin $D_3$, 100 mg Vitamin E, 50 mg $MnSO_4 \times H_2O$, 30 mg $ZnSO_4 \times 7H_2O$ in Getreidemehl als Trägerstoff.

Der Wirkstoff-Prämix wird der Vitamin-Mineral-Mischung in der erforderlichen Menge beigemischt und diese anschließend sorgfältig mit den übrigen Bestandteilen vermischt.

## Beispiel

Ratten-Fütterungsversuch

Weibliche Laborratten 90-110 g schwer vom Typ SPF Wistar (Züchtung Hagemann) werden ad lib mit Standard Rattenfutter, das mit der gewünschten Menge Wirkstoff versetzt ist, gefüttert. Jeder Versuchsansatz wird mit Futter der identischen Charge durchgeführt, so daß Unterschiede in der Zusammensetzung des Futters die Vergleichbarkeit der Ergebnisse nicht beeinträchtigen können.

Die Ratten erhalten Wasser ab lib.

Jeweils 12 Ratten bilden eine Versuchsgruppe und werden mit Futter, das mit der gewünschten Menge Wirkstoff versetzt ist, gefüttert. Eine Kontrollgruppe erhält Futter ohne Wirkstoff. Die Versuchsgruppen werden so zusammengestellt, daß das durchschnittliche Körpergewicht sowie die Streuung in den Körpergewichten der Ratten in jeder Versuchsgruppe gleich ist, so daß eine Vergleichbarkeit der Versuchsgruppen untereinander gewährleistet ist.

Die Tiere werden vor Versuchsbeginn 2 Tage an die neuen Haltungsbedingungen adaptiert, während dieser Zeit wird Futter ohne Wirkstoffzusatz gegeben. Danach erhalten die Tiere 13 Tage lang Futter, das Wirkstoff enthält. Es wird die relative Gewichtszunahme im Verhältnis zur unbehandelten Kontrolle bestimmt.

Es werden die aus der Tabelle ersichtlichen Ergebnisse erhalten:

Tabelle

| Ratten-Fütterungsversuch | | |
|---|---|---|
| Wirkstoff Bsp-Nr. | Wirkstoffkonzentration [ppm] | relative Gewichtszunahme [%] |
| 1 | 50 | 25 |
| 2 | 50 | 11 |
| 3 | 50 | 22 |

Beispiele

1. 1-(5-Benzimidazoyl)-2-tert.butylaminoethanol

Herstellung nach Verfahren 3a):

Zur Lösung von 2,5 g (34 mmol) tert.Butylamin in 50 ml Methanol werden bei Raumtemperatur 1,5 g (4,7 mmol) 5-Benzimidazolyl-brommethylketonhydrobromid auf einmal zugegeben, auf etwa 40 °C erwärmt und 30 min. gerührt. Dann wird auf 0 °C abgekühlt, 300 mg (7,9 mmol) $NaBH_4$ zugegeben und 30 min. gerührt. Dann wird eingedampft, zwischen Essigester und Wasser verteilt, die organische Phase abgetrennt, über $Na_2SO_4$ getrocknet und eingedampft.
Ausbeute: 950 mg (87 %); Fp. 70 °C

Herstellung nach Verfahren 3d):

500 mg (2,3 mmol) 5-Benzimidazolylglyoxal und 730 mg (10 mmol) tert. Butylamin werden in 50 ml abs. Methanol nach Zugabe von etwas Molsieb 12 h bei Raumtemperatur gerührt. Dann wird auf 0 °C abgekühlt und 180 mg (5 mmol) $NaBH_4$ zugegeben. Nach 30 min. wird eingedampft und zwischen Essigester und Wasser verteilt. Die organische Phase wird abgetrennt, über $Na_2SO_4$ getrocknet und eingedampft.
Ausbeute: 375 mg (70 %), Fp. 70 °C

2. 1-(4-Brom-6-benzimidazolyl)-2-tert.butylaminoethanol

hergestellt nach Verfahren 3d)
Ausbeute: 71 %; Fp. 68 °C

3. 1-(5-Benzimidazoyl)-2-(2-[3-[4-(2-hydroxyethoxyphenyl]-propyl]amino)ethanol

hergestellt nach Verfahren 3d)
Ausbeute: 70 %, Fp. 112 °C

4. 1-(2-Hydroxy-5-benzimidazolyl)-2-tert.butylaminoethanol

hergestellt nach Verfahren 3a)
Ausbeute: 75 %, Fp. 85 °C

5. 5-Acetylbenzimidazol

Eine Mischung von 2 g (13,3 mmol) 3,4-Diaminoacetophenon, 0,92 g (20 mmol) Ameisensäure und 15 ml 4N Salzsäure wird zum Rückfluß erhitzt. Nach 15 Minuten wird abgekühlt, mit 50 ml Wasser verdünnt und dreimal mit je 25 ml Essigester gewaschen. Die wäßrige Phase wird mit konz. Ammoniak alkalisch gestellt und dreimal mit je 25 ml Essigester extrahiert. Die vereinigten organischen Phasen werden über $Na_2SO_4$ getrocknet und eingedampft. Es verbleiben 2 g (94 % d.Th.) eines rotbraunen Öls. $^1$H-NMR (DMSO-$d_6$, δ(ppm)): 2,65 (s,3H); 7,65 (m,1H); 7,85 (d,1H); 8,3 (m,1H); 8,45 (s,1H); 12,8 (s,1H).

6. 5-Bromacetly)benzimidazol-hydrobromid

Zur Lösung von 1,2 g 97,5 mmol) 5-Acetylbenzimidazol und 1,35 g 48 %iger wäßriger HBr (≙ 8 mmol HBr) in 15 ml Eisessig werden bei Raumtemperatur 1,21 g (7,6 mmol) Brom in 5 ml Eisessig gegeben. Die Mischung wird 30 Minuten bei 40 °C gerührt, abgekühlt und das ausgefallene Produkt abgesaugt.
Ausbeute: 1,5 g (63 % d.Th.), Fp. >250 °C. $^1$H-NMR ($CD_3OD$, δ(ppm)): 4,8 (s,2H); 7,9 (dd,1H); 8,2 (dd,1H); 8,5 (d,1H); 9.4 (s,1H).

**Ansprüche**

1. Verwendung von Benzimidazolderivaten der Formel I

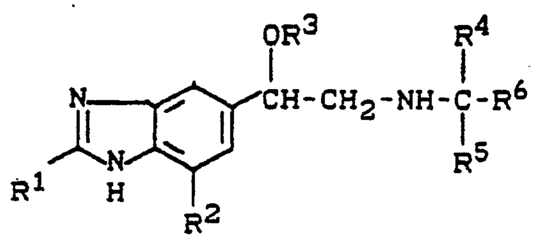

(I)

in welcher
$R^1$ für Wasserstoff, Hydroxy, Alkyl, Halogenalkyl steht,
$R^2$ für Wasserstoff, Alkyl, Halogen, CN, Halogenalkyl steht,
$R^3$ für Wasserstoff, Alkyl, Acyl steht,
$R^4$ für Wasserstoff, Alkyl, Halogenalkyl steht,
$R^5$ für Alkyl, Halogenalkyl steht,
$R^6$ für Alkyl, Cycloalkyl, gegebenenfalls substituiert durch Halogen, Cycloalkyl, OH, Alkoxy, Halogenalkoxy, Aryl, das seinerseits substituiert sein kann durch Alkyl, Halogen, CN oder die Reste -$COR^7$, -O-Alkylen-$COR^7$, -Alkylen-$R^8$ oder -O-Alkylen-$R^8$, steht,
$R^7$ für Hydroxy, Alkoxy oder -$NR^9R^{10}$ steht,
$R^8$ für Hydroxy, Alkoxy oder -$NR^9R^{10}$ steht,
$R^9$ für Wasserstoff, Alkyl steht,
$R^{10}$ für Wasserstoff, Alkyl steht
sowie deren Tautomere and physiologisch verträgliche Salze zur Förderung der Leistung und des Wachstums von Tieren.

2. Mittel zur Leistungsförderung von Tieren, gekennzeichnet durch einen Gehalt an Benzimidazolderivaten der Formel I gemäß Anspruch 1.

3. Tierfutter, Trinkwasser für Tiere, Zusätze für Tierfutter und Trinkwasser für Tiere, gekennzeichnet durch einen Gehalt an Benzimidazolderivaten der Formel I gemäß Anspruch 1.

4. Verwendung von Benzimidazolderivaten der Formel (I) gemäß Anspruch 1 zur Herstellung von Mitteln zur Leistungsförderung bei Tieren.

5. Benzimidazolderivate der Formel I

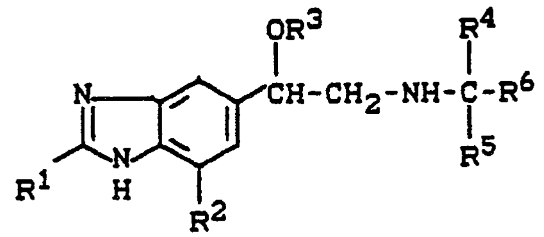

(I)

in welcher

$R^1$ für Wasserstoff, Hydroxy, Alkyl, Halogenalkyl steht,

$R^2$ für Wasserstoff, Alkyl, Halogen, CN, Halogenalkyl steht,

$R^3$ für Wasserstoff, Alkyl, Acyl steht,

$R^4$ für Wasserstoff, Alkyl, Halogenalkyl steht,

$R^5$ für Alkyl, Halogenalkyl steht,

$R^6$ für Alkyl, Cycloalkyl, gegebenenfalls substituiert durch Halogen, Cycloalkyl, OH, Alkoxy, Halogenalkoxy, Aryl, das seinerseits substituiert sein kann durch Alkyl, Halogen, CN oder die Reste $-COR^7$, $-O-Alkylen-COR^7$, $-Alkylen-R^8$ oder $-O-Alkylen-R^8$, steht,

$R^7$ für Hydroxy, Alkoxy oder $-NR^9R^{10}$ steht,

$R^8$ für Hydroxy, Alkoxy oder $-NR^9R^{10}$ steht,

$R^9$ für Wasserstoff, Alkyl steht,

$R^{10}$ für Wasserstoff, Alkyl steht

sowie deren Tautomere and physiologisch verträgliche Salze, ausgenommen Verbindungen in denen

$R^1$ für Wasserstoff steht,

$R^4$ für Wasserstoff steht,

$R^5$ für Methyl steht,

$R^6$ für Methyl steht,

sowie deren Tautomere and Salze.

6. Verfahren zur Herstellung von Benzimidazolderivate der Formel I

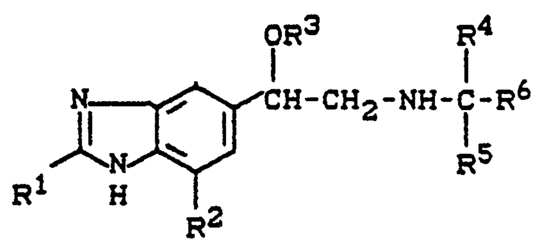

(I)

in welcher

$R^1$ für Wasserstoff, Hydroxy, Alkyl, Halogenalkyl steht,

$R^2$ für Wasserstoff, Alkyl, Halogen, CN, Halogenalkyl steht,

$R^3$ für Wasserstoff, Alkyl, Acyl steht,

$R^4$ für Wasserstoff, Alkyl, Halogenalkyl steht,

$R^5$ für Alkyl, Halogenalkyl steht,

$R^6$ für Alkyl, Cycloalkyl, gegebenenfalls substituiert durch Halogen, Cycloalkyl, OH, Alkoxy, Halogenalkoxy, Aryl, das seinerseits substituiert sein kann durch Alkyl, Halogen, CN oder die Reste $-COR^7$, $-O-Alkylen-COR^7$, $-Alkylen-R^8$ oder $-O-Alkylen-R^8$, steht,

$R^7$ für Hydroxy, Alkoxy oder $-NR^9R^{10}$ steht,

$R^8$ für Hydroxy, Alkoxy oder $-NR^9R^{10}$ steht,

$R^9$ für Wasserstoff, Alkyl steht,

$R^{10}$ für Wasserstoff, Alkyl steht
sowie deren Tautomere and physiologisch verträgliche Salze, ausgenommen Verbindungen in denen $R^1$ und $R^4$ für Wasserstoff sowie $R^5$ und $R^6$ für Methyl stehen,
dadurch gekennzeichnet, daß man

a) Halogenmethylketone der Formel II

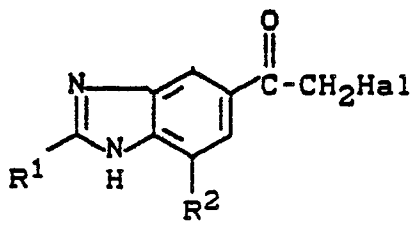

(II)

in welcher
$R^1$ und $R^2$ die oben angegebene Bedeutung haben und
Hal für Halogen steht,
mit Aminen der Formel III

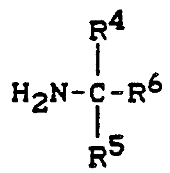

(III)

in welcher
$R^4$ bis $R^6$ die oben angegebene Bedeutung haben,
umsetzt und anschließend die Carbonylgruppe reduziert oder

b) $\beta$-Halogenethylverbindungen der Formel IV

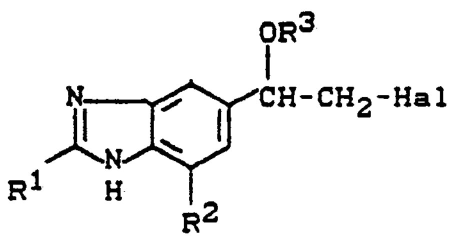

(IV)

in welcher
$R^1$ und $R^3$ die oben angegebene Bedeutung haben und
Hal für Halogen steht,
mit Aminen der Formel III

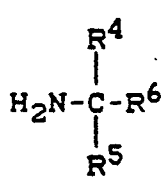

(III)

in welcher
$R^4$ bis $R^6$ die oben angegebene Bedeutung haben
umsetzt oder

c) für den Fall, daß in Formel I $R^4$ für Wasserstoff steht, indem man Verbindungen der Formel V

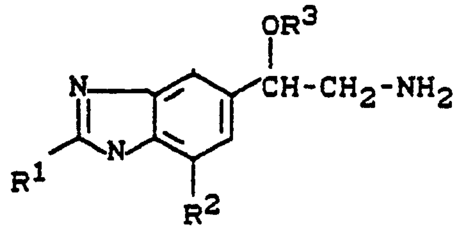

(V)

in welcher
$R^1$ und $R^3$ die oben angegebene Bedeutung haben,
mit Verbindungen der Formel VI

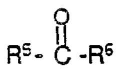

(VI)

in welcher
$R^5$, $R^6$ die oben angegebene Bedeutung haben, unter reduzierenden Bedingungen umsetzt oder

d) Verbindungen der Formel VII

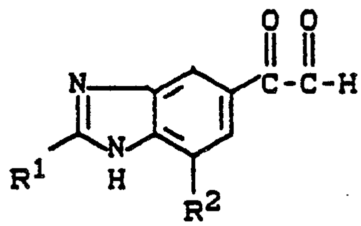

(VII)

in welcher
$R^1$ und $R^2$ die oben angegebene Bedeutung haben,
mit Aminen der Formel III

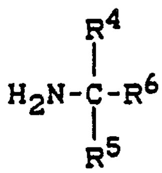

(III)

in welcher
$R^4$ bis $R^6$ die oben angegebene Bedeutung haben, unter reduzierenden Bedingungen umsetzt.

7. Verbindungen der Formel II

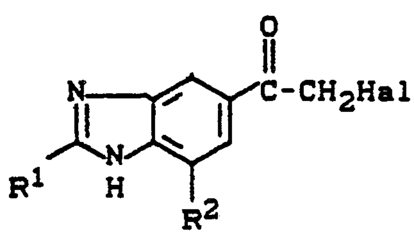

(II)

in welcher
$R^1$ für Wasserstoff
$R^2$ für Wasserstoff, Alkyl, Halogen
Hal für Halogen steht.

8. Verfahren zur Herstellung der Verbindungen der Formel II gemäß Anspruch 7, dadurch gekennzeichnet, daß man Acetylverbindungen der Formel VIII

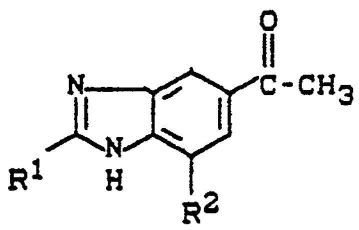

(VIII)

in welcher
$R^1$ für Wasserstoff,
$R^2$ für Wasserstoff, Alkyl, Halogen steht,
a) mit elementarem Halogen umsetzt, oder
b) mit Kupferhalogeniden der Formel $CuHal_2$ umsetzt.

9. Neue Verbindungen der Formel VIII

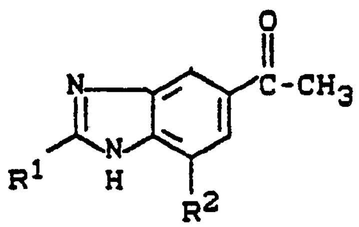

(VIII)

in welcher
$R^1$ für Wasserstoff,
$R^2$ für Wasserstoff, Alkyl, Halogen steht.

10. Verfahren zur Herstellung der Verbindungen der Formel VIII gemäß Anspruch 9, dadurch gekennzeichnet, daß man 2,4-Diaminoacetophenone der Formel IX

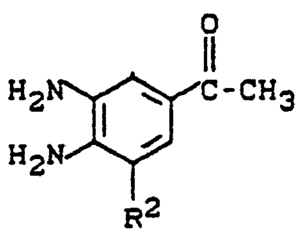

IX

in welcher
$R^2$ für Wasserstoff, Alkyl oder Halogen steht,
mit Ameisensäure in Gegenwart anorganischer Säuren umsetzt.

11. Neue Verbindungen der Formel IV

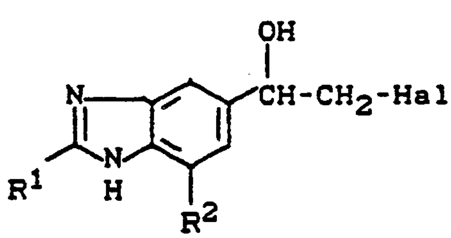

(IV)

in welcher
$R^1$ für Wasserstoff, Hydroxy, Alkyl, Halogenalkyl steht,
$R^2$ für Wasserstoff, Alkyl, Halogen, CN, Halogenalkyl steht.

12. Verfahren zur Herstellung der Verbindungen der Formel IV gemäß Anspruch 11, dadurch gekennzeichnet, daß man Verbindungen der Formel II

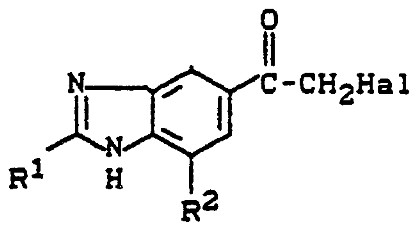

(II)

in welcher
$R^1$ und $R^2$ die bei (8) angegebenen Bedeutungen haben, reduziert.

13. Neue Verbindungen der Formel V

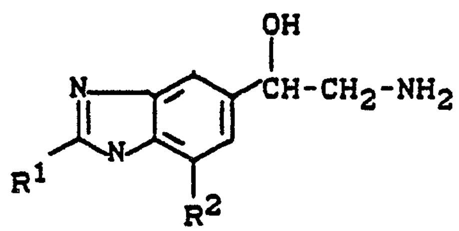

(V)

in welcher
$R^1$ für Wasserstoff, Alkyl, Halogenalkyl steht,
$R^2$ für Alkyl, Halogen, CN, Halogenalkyl steht.

14. Verfahren zur Herstellung der Verbindungen der Formel V gemäß Anspruch 13, dadurch gekennzeichnet, daß man Verbindungen der Formel X

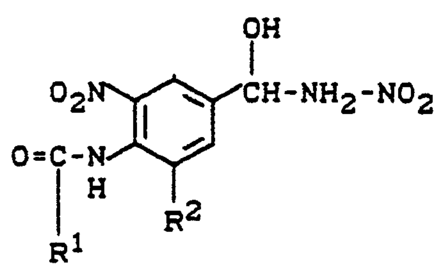

(X)

in welcher
$R^1$ und $R^2$ die in Anspruch 13 angegebene Bedeutung haben,
in Gegenwart von Katalysatoren mit Wasserstoff reduziert.

15. Neue Verbindungen der Formel VII

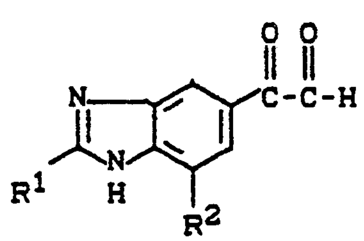

(VII)

in welcher
$R^1$ für Wasserstoff, Hydroxy, Alkyl, Halogenalkyl steht,
$R^2$ für Wasserstoff, Alkyl, Halogen, CN, Halogenalkyl steht.

16. Verfahren zur Herstellung der Verbindungen der Formel VII gemäß Anspruch 15, dadurch gekennzeichnet, daß man

a) Verbindungen der Formel II

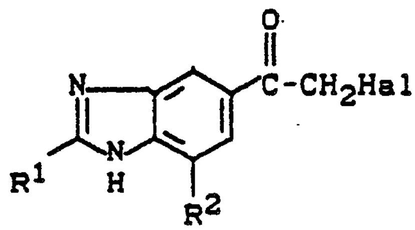

(II)

in welcher
$R^1$ und $R^2$ die oben angegebenen Bedeutung haben und
Hal für Halogen steht
oder

b) Verbindungen der Formel VIII

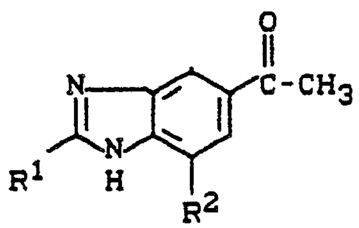

(VIII)

in welcher
$R^1$ und $R^2$ die in Anspruch 15 angegebene Bedeutung haben, oxidiert.

Europäisches Patentamt

# EUROPÄISCHER RECHERCHENBERICHT

Nummer der Anmeldung

EP 88 12 1880

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | FR-M- 3 697 (IMPERIAL CHEMICAL INDUSTRIES) * Seiten 1-4 * | 5-7,11, 13,15 | C 07 D 235/06<br>C 07 D 235/08<br>C 07 D 235/26<br>C 07 D 235/10<br>A 61 K 31/415 |
| X | JOURNAL OF MEDICINAL CHEMISTRY, Band 15, Nr. 1, 1. Januar 1972, Seiten 49-57, Columbus, Ohio, US; M.S. CHODNEKAR et al.: "Beta-adrenergic blocking agents. 11. Heterocyclic analogs of pronethalol [2-isopropylamino-1-(2-naphtyl)ethanol]" * Seite 50; Figuren compound, Nr. 6; Seite 56, Spalte 1, Absatz D * | 5 | |
| A | JOURNAL OF MEDICINAL CHEMISTRY, Band 21, Nr. 1, 1. Januar 1978, Seiten 72-78, American Chemical Society, Columbus, Ohio, US; C.D. ARNETT et al.: "Synthesis and adrenergic activity of benzimidazole bioisosteres of norepinephrine and isoproterenol" | | **RECHERCHIERTE SACHGEBIETE (Int. Cl.4)** |
| D,A | GB-A-1 559 915 (MERCK & CO.) | | C 07 D 235/00<br>A 61 K 31/00 |

Der vorliegende Recherchenbericht wurde für alle Patentansprüche erstellt

| Recherchenort | Abschlußdatum der Recherche | Prüfer |
|---|---|---|
| DEN HAAG | 11-04-1989 | DE BUYSER I.A.F. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)